# EUROPEAN PATENT APPLICATION

(11) **EP 4 431 097 A1**
(43) Date of publication of application: **18.09.2024**
(21) Application number: 22892074.0
(22) Date of filing: 11.11.2022
(51) Int. Cl.: A61K 31/498, A61K 31/4709, A61P 7/04, A61P 37/00

(54) **USE OF QUINOLINONE DERIVATIVE IN TREATMENT OF IMMUNE THROMBOCYTOPENIA**

(30) Priority: 12.11.2021 CN 202111340688
(71) Applicant: Chia Tai Tianqing Pharmaceutical Group Co., Ltd., Lianyungang, Jiangsu 222062 (CN)
(72) Inventor: ZHAO, Qian, Lianyungang, Jiangsu 222062 (CN); WANG, Xunqiang, Lianyungang, Jiangsu 222062 (CN); ZHANG, Xiquan, Lianyungang, Jiangsu 222062 (CN); YU, Ding, Lianyungang, Jiangsu 222062 (CN)
(74) Representative: v. Bezold & Partner Patentanwälte - PartG mbB
(86) International application number: PCT/CN2022/131278
(87) International publication number: WO 2023/083281

(57) **Abstract**

The present invention relates to a use of a quinolinone derivative in treatment of immune thrombocytopenia, and in particular to a use of a compound of formula (I) or a pharmaceutically-acceptable salt or pharmaceutical composition thereof in treatment of immune thrombocytopenia.

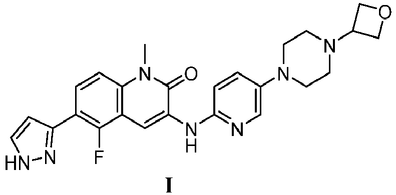

## Description

### CROSS-REFERENCE TO RELATED APPLICATIONS

The present application claims the benefit of and priority to Chinese Patent Application No. 202111340688.4 filed with China National Intellectual Property Administration on November 12, 2021, which is incorporated herein by reference in its entirety.

### TECHNICAL FIELD

The present application belongs to the field of medical chemistry and relates to use of a quinolinone derivative in treating immune thrombocytopenia and in particular to use of a compound of formula I or a pharmaceutically acceptable salt thereof in treating immune thrombocytopenia.

### BACKGROUND

Primary immune thrombocytopenia (ITP) is an acquired autoimmune hemorrhagic disease mainly characterized by an isolated decrease in peripheral blood platelet count without clear causes. At present, there is still no population-based epidemiological data on immune thrombocytopenia in China, and the annual morbidity of adult immune thrombocytopenia reported abroad is about (2-10)/100,000. Immune thrombocytopenia may occur in any age group and is more commonly seen in older people over 60 and in women of childbearing age. The clinical manifestation of immune thrombocytopenia varies in patients. Patients may be asymptomatic or only experience cutaneous mucosal bleeding. About 5% of patients with immune thrombocytopenia experience life-threatening hemorrhages in vital organs and intracranial hemorrhages, and about 15% of patients with immune thrombocytopenia are hospitalized for a hemorrhagic event within 5 years after diagnosis. In addition to hemorrhagic events, the vast majority of patients with immune thrombocytopenia experience fatigue and a decline in health-related quality of life (HRQoL).

Spleen tyrosine kinase (Syk), an intracellular tyrosine protein kinase, is a member of the ZAP70 protein kinase family. Syk is involved in the FcR signaling pathway. Upon the binding of FcR to its ligand, receptor ligation results in the autophosphorylation of Syk. Thus, Syk recruitment and downstream signaling cascade activation will lead to cytoskeletal rearrangement and phagocytosis. Syk plays a role in various immune recognition receptors, and its sustained expression has been implicated in the development and progression of a variety of hematologic malignancies. Therefore, Syk inhibitors can be used to treat allergic, autoimmune, and inflammatory diseases as well as some hematologic malignancies.

WO2018228475 discloses a series of compounds that act as Syk inhibitors and also specifically discloses compounds of formula I of the following structure:

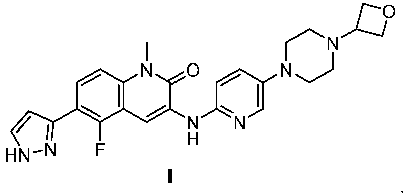

### SUMMARY

In one aspect, the present application provides a compound of formula I or a pharmaceutically acceptable salt thereof for use in treating or preventing immune thrombocytopenia in a patient:

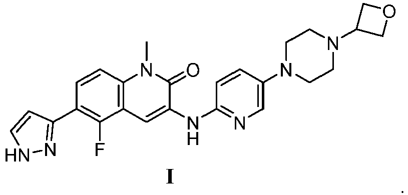

In another aspect, the present application provides a pharmaceutical composition for use in treating or preventing immune thrombocytopenia in a patient, the pharmaceutical composition comprising the compound of formula I or the pharmaceutically acceptable salt thereof.

In some embodiments, the pharmaceutical composition further comprises a pharmaceutically acceptable carrier and/or excipient.

In some embodiments, the pharmaceutical composition of the present application comprises the compound of formula I or the pharmaceutically acceptable salt thereof as a single active agent.

In some embodiments, the pharmaceutical composition of the present application comprises a therapeutically effective amount or a prophylactically effective amount of the compound of formula I or the pharmaceutically acceptable salt thereof.

In some embodiments, the pharmaceutical composition of the present application comprises a single dose of 10-200 mg (calculated based on the weight of the compound of formula I) of the compound of formula I or the pharmaceutically acceptable salt thereof.

In some embodiments, the pharmaceutical composition of the present application comprises 10-200 mg (calculated based on the weight of the compound of formula I) of the compound of formula I or the pharmaceutically acceptable salt thereof as a single active agent.

In another aspect, the present application provides use of the compound of formula I or the pharmaceutically acceptable salt thereof, or the pharmaceutical composition thereof for preparing a medicament for treating or preventing immune thrombocytopenia in a patient.

In another aspect, the present application provides use of the compound of formula I or the pharmaceutically acceptable salt thereof, or the pharmaceutical composition thereof in treating or preventing immune thrombocytopenia in a patient.

In another aspect, the present application provides a method for treating or preventing immune thrombocytopenia in a subject, the method comprising administering to a subject in need thereof a therapeutically effective amount or a prophylactically effective amount of the compound of formula I or the pharmaceutically acceptable salt thereof, or the pharmaceutical composition thereof.

In some embodiments of the present application, the compound of formula I or the pharmaceutically acceptable salt thereof of the present application is used as a single active agent.

In some embodiments, the compound of formula I or the pharmaceutically acceptable salt thereof of the present application is used in a single dose of 10-200 mg (calculated based on the weight of the compound of formula I).

In some embodiments of the present application, the compound of formula I or the pharmaceutically acceptable salt thereof of the present application may be in the form of a pharmaceutical composition comprising a therapeutically effective amount or a prophylactically effective amount of the compound of formula I or the pharmaceutically acceptable salt thereof.

In another aspect, the present application provides a kit for use in treating immune thrombocytopenia in a patient, the kit comprising the compound of formula I or the pharmaceutically acceptable salt thereof, or the pharmaceutical composition thereof, and instructions for use in treating or preventing immune thrombocytopenia in the patient.

In some embodiments, the compound of formula I or the pharmaceutically acceptable salt thereof of the present application may be in the form of a single dose of 10-200 mg (calculated based on the weight of the compound of formula I).

In some embodiments, the pharmaceutical composition may be a single-dose pharmaceutical composition comprising 10-200 mg (calculated based on the weight of the compound of formula I) of the compound of formula I or the pharmaceutically acceptable salt thereof as an active ingredient.

### Compound of Formula I or Pharmaceutically Acceptable Salt Thereof

In some embodiments of the present application, the compound of formula I of the present application may be obtained by reference to a preparation method described in WO2018228475 or WO2020119785.

As used in the present application, the pharmaceutically acceptable salt of the compound of formula I may be a hydrochloride of the compound of formula I, e.g., the monohydrochloride of the compound of formula I. The dose of the compound of formula I or the pharmaceutically acceptable salt thereof referred to in the present application is calculated based on the molecular weight of the compound of formula I, unless otherwise stated.

In some embodiments of the present application, the hydrochloride of the compound of formula I of the present application may be obtained by reference to a preparation method described in WO2020119785.

### Pharmaceutical Composition of Compound of Formula I or Pharmaceutically Acceptable Salt Thereof

In some embodiments of the present application, the compound of formula I or the pharmaceutically acceptable salt thereof is a pharmaceutical composition comprising a therapeutically effective amount or a prophylactically effective amount of the compound of formula I or the pharmaceutically acceptable salt thereof.

In some embodiments of the present application, the pharmaceutical composition comprises a single dose of 10-200 mg of the compound of formula I or the pharmaceutically acceptable salt thereof. In some embodiments, the pharmaceutical composition comprises a single dose of 10 mg, 20 mg, 40 mg, 50 mg, 60 mg, 80 mg, 100 mg, 120 mg, 150 mg, or 200 mg, or a range formed with any two of the foregoing values as endpoints or any value therein of the compound of formula I or the pharmaceutically acceptable salt thereof, e.g., 50 mg or 200 mg. In some embodiments, the pharmaceutical composition comprises a single dose of 200 mg of the compound of formula I or the pharmaceutically acceptable salt thereof.

In some embodiments of the present application, the pharmaceutical composition is a single-dose pharmaceutical composition. In some embodiments of the present application, the pharmaceutical composition is a pharmaceutical composition of a single dose of 10-200 mg of the compound of formula I or the pharmaceutically acceptable salt thereof.

In some embodiments of the present application, the compound of formula I or the pharmaceutically acceptable salt thereof is administered daily according to a treatment cycle of 2-6 weeks (e.g., 28 days), with the total dose per treatment cycle comprising 0.7 g-42 g, e.g., 5.6-22.4 g. In some embodiments, the total dose per treatment cycle of the compound of formula I or the pharmaceutically acceptable salt thereof is selected from the group consisting of 5.6 g, 11.2 g, 16.8 g, 22.4 g, and a range formed by any of the aforementioned values. In some embodiments, the total dose per treatment cycle of the compound of formula I or the pharmaceutically acceptable salt thereof preferably comprises 11.2-22.4 g. In some embodiments, the total dose per treatment cycle of the compound of formula I or the pharmaceutically acceptable salt thereof preferably comprises 11.2 g or 16.8 g. In some embodiments, the total dose per treatment cycle of the compound of formula I or the pharmaceutically acceptable salt thereof preferably comprises 16.8 g.

In some embodiments of the present application, the pharmaceutical composition includes, but is not limited to, formulations suitable for oral, parenteral, or local administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, a tablet and a capsule.

In some embodiments of the present application, the pharmaceutical composition is a solid pharmaceutical composition.

In some embodiments of the present application, the pharmaceutical composition is a tablet.

In some embodiments of the present application, the pharmaceutical composition of the compound of formula I or the pharmaceutically acceptable salt thereof may be a solid pharmaceutical composition of a hydrochloride of the compound of formula I.

In some embodiments, the pharmaceutical composition of the present application may be a pharmaceutical composition of the monohydrochloride of the compound of formula I. In some embodiments, the pharmaceutical composition of the present application may be a solid pharmaceutical composition of the monohydrochloride of the compound of formula I.

In some embodiments of the present application, the pharmaceutical composition of the compound of formula I or the pharmaceutically acceptable salt thereof is a tablet of a hydrochloride of the compound of formula I, e.g., the monohydrochloride of the compound of formula I.

The pharmaceutical composition of the compound of formula I or the pharmaceutically acceptable salt thereof may further comprise a pharmaceutically acceptable carrier and/or excipient. The pharmaceutically acceptable carrier and/or excipient is well known to those skilled in the art and includes, for example, fillers, absorbents, wetting agents, binders, disintegrants, lubricants, and the like.

The pharmaceutical composition of the compound of formula I or the pharmaceutically acceptable salt thereof of the present application can be manufactured using methods well known in the art, such as conventional mixing, dissolution, granulation, dragee-making, levigation, emulsifying, lyophilization, and the like.

A solid oral composition can be prepared by conventional mixing, filling, or tableting. For example, the solid oral composition can be obtained by the following method: mixing the active compound with solid auxiliary materials, optionally grinding the resulting mixture, adding additional suitable excipients if desired, and processing the mixture into granules to get the core parts of tablets or capsules or dragees. Suitable auxiliary materials include, but are not limited to: binders, diluents, disintegrants, lubricants, glidants, sweeteners, flavoring agents, or the like.

### Immune Thrombocvtopenia

In some embodiments of the present application, the immune thrombocytopenia is primary immune thrombocytopenia or secondary immune thrombocytopenia. Preferably, the immune thrombocytopenia is primary immune thrombocytopenia.

In some embodiments of the present application, the immune thrombocytopenia includes: newly diagnosed immune thrombocytopenia, persistent immune thrombocytopenia, or chronic immune thrombocytopenia. Preferably, the immune thrombocytopenia is persistent immune thrombocytopenia or chronic immune thrombocytopenia. Further preferably, the immune thrombocytopenia is chronic immune thrombocytopenia.

In some embodiments of the present application, the immune thrombocytopenia is persistent or chronic primary immune thrombocytopenia.

In some embodiments of the present application, the immune thrombocytopenia is adult persistent or chronic primary immune thrombocytopenia.

In some embodiments of the present application, the immune thrombocytopenia is recurrent and/or refractory primary immune thrombocytopenia.

In some embodiments of the present application, the immune thrombocytopenia is recurrent and/or refractory persistent primary immune thrombocytopenia.

In some embodiments of the present application, the immune thrombocytopenia is recurrent and/or refractory chronic primary immune thrombocytopenia.

In some embodiments of the present application, the immune thrombocytopenia is adult recurrent and/or refractory persistent primary immune thrombocytopenia.

In some embodiments of the present application, the immune thrombocytopenia is adult recurrent and/or refractory chronic primary immune thrombocytopenia.

In some embodiments of the present application, a patient with the persistent or chronic immune thrombocytopenia is a patient who has been undergoing a persistent decrease in platelet count for more than 3 months following diagnosis.

In some embodiments of the present application, a patient with the persistent or chronic immune thrombocytopenia is a patient who has been undergoing a persistent decrease in platelet count for more than 6 months following diagnosis.

In some embodiments of the present application, a patient with the persistent or chronic immune thrombocytopenia has a WHO bleeding score of 0-1.

In some embodiments of the present application, a patient with the persistent or chronic immune thrombocytopenia has a mean value of at least two independent platelet counts prior to treatment of less than 30 × 10⁹/L, with each of the platelet counts being no greater than 35 × 10⁹/L.

In some embodiments of the present application, a patient with the persistent or chronic immune thrombocytopenia has a mean value of at least two independent platelet counts prior to treatment of less than 30 × 10⁹/L, with each of the platelet counts being no greater than 35 × 10⁹/L, and the patient experienced no severe bleeding within 4 weeks prior to treatment. In some embodiments of the present application, the severe bleeding refers to a WHO bleeding score of ≥3.

In some embodiments of the present application, a patient with the immune thrombocytopenia is a patient who has previously received one or more prior therapies for immune thrombocytopenia. In some embodiments, a patient with the immune thrombocytopenia is a patient who has previously received one, two, three, four, or five prior therapies for immune thrombocytopenia.

In some embodiments of the present application, the prior therapies for immune thrombocytopenia include a drug therapy or a surgical therapy, or a combination thereof.

In some embodiments of the present application, the drug therapy includes, but is not limited to, a glucocorticoid therapy, an immunoglobulin therapy, a molecularly targeted therapy, a thrombopoietic drug therapy, or other drug therapies that can be used for treating immune thrombocytopenia.

In some embodiments of the present application, the glucocorticoid includes, but is not limited to, prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, or dexamethasone. Preferably, the glucocorticoid includes dexamethasone and prednisone. In some embodiments, the glucocorticoid therapy may be a pulsed high-dose glucocorticoid therapy.

In some embodiments of the present application, the immunoglobulin includes an immunoglobulin for injection. In some embodiments of the present application, the immunoglobulin may be a gamma immunoglobulin. Preferably, the immunoglobulin for injection is a gamma immunoglobulin for injection, e.g., a gamma immunoglobulin for intravenous injection.

In some embodiments of the present application, the drug for molecularly targeted therapy includes, but is not limited to, rituximab, veltuzumab, daclizumab, basiliximab, alemtuzumab, or ocrelizumab. Preferably, the drug for molecularly targeted therapy includes rituximab.

In some embodiments of the present application, the thrombopoietic drug includes, but is not limited to, avatrombopag, eltrombpag, romiplostim, lusutrombopag, or recombinant human thrombopoietin (rhTPO). Preferably, the thrombopoietic drug includes avatrombopag, eltrombpag, romiplostim, or recombinant human thrombopoietin (rhTPO). Further preferably, the thrombopoietic drug includes eltrombpag and recombinant human thrombopoietin.

In some embodiments of the present application, the other drugs that can be used for treating immune thrombocytopenia include, but are not limited to, immunosuppressants. In some embodiments of the present application, the other drugs that can be used for treating immune thrombocytopenia include, but are not limited to, immunosuppressants such as ganciclovir sodium, imatinib, cyclosporine (A), mycophenolate sodium, mycophenolate mofetil, danazol, azathioprine, psoralen, methotrexate, hydroxychloroquine, clofazimine, cyclophosphamide, thalidomide, azithromycin, montelukast, sorafenib, ruxolitinib, decitabine, vincristine, alefacept, or all-trans retinoic acid. Preferably, the other drugs that can be used for treating immune thrombocytopenia include danazol, azathioprine, cyclosporine (A), vincristine, all-trans retinoic acid, or mycophenolate mofetil. Further preferably, the other drugs that can be used for treating immune thrombocytopenia include danazol, azathioprine, cyclosporine (A), all-trans retinoic acid, or vincristine. The other drug therapies that can be used for treating immune thrombocytopenia include an all-trans retinoic acid and danazol combination therapy, a decitabine therapy, a cyclosporine (A) therapy, an azathioprine therapy, a danazol therapy, and/or a vincristine therapy.

In some embodiments of the present application, the drug used for the drug therapy includes, but is not limited to, prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, immunoglobulins for injection (e.g., a gamma immunoglobulin for injection), rituximab, veltuzumab, daclizumab, basiliximab, ocrelizumab, alemtuzumab, avatrombopag, eltrombpag, romiplostim, lusutrombopag, recombinant human thrombopoietin (rhTPO), danazol, azathioprine, cyclosporine (A), mycophenolate mofetil, vincristine, all-trans retinoic acid, or decitabine, or a combination of one or more of the drugs described above.

In some embodiments of the present application, the surgical therapy is a splenectomy therapy.

In some embodiments of the present application, a patient with the immune thrombocytopenia may be a patient who has not benefited much from splenectomy or has suffered from a postoperative recurrence.

In some embodiments of the present application, a patient with the immune thrombocytopenia may be a patient who has previously failed one or more first-line or multi-line standard therapies. In some embodiments, a patient with the immune thrombocytopenia may be a patient who has previously failed one or more first-line, second-line, third-line, or fourth-line standard therapies. In some embodiments, a patient with the immune thrombocytopenia may be a patient who has previously failed one or more first-line, second-line, or third-line standard therapies. In some embodiments, a patient with the immune thrombocytopenia may be a patient who has previously failed one, two, three, four, or five first-line standard therapies.

In some embodiments of the present application, the first-line standard therapies include, but are not limited to, a glucocorticoid therapy and/or an immunoglobulin-for-injection therapy. In some embodiments, the glucocorticoid therapy is a dexamethasone or prednisone therapy. In some embodiments, the immunoglobulin-for-injection therapy is a gamma-globulin-for-injection therapy.

In some embodiments of the present application, the second-line standard therapies include, but are not limited to, a thrombopoietic drug therapy and/or a molecularly targeted therapy and/or a surgical therapy. In some embodiments, the thrombopoietic drug therapy is an eltrombpag and/or recombinant human thrombopoietin therapy. In some embodiments, the molecularly targeted therapy is a rituximab therapy. In some embodiments, the surgical therapy is a splenectomy therapy.

In some embodiments of the present application, the third-line standard therapies include, but are not limited to, an all-trans retinoic acid and danazol combination therapy and/or a decitabine therapy.

In some embodiments of the present application, the fourth-line standard therapies include, but are not limited to, an azathioprine and/or cyclosporine (A) and/or danazol and/or vinca alkaloid therapy.

In some embodiments of the present application, a patient with the immune thrombocytopenia may be a patient who has previously failed at least one first-line standard therapy for immune thrombocytopenia (e.g., a glucocorticoid therapy and/or a gamma-globulin-for-intravenous-injection therapy), or has not benefited much from splenectomy or has suffered from a postoperative recurrence.

In some embodiments of the present application, a patient with the immune thrombocytopenia may be a patient who was diagnosed with adult persistent or chronic immune thrombocytopenia more than 3 months ago, and/or has previously failed at least one first-line standard therapy for immune thrombocytopenia (e.g., a glucocorticoid therapy and/or a gamma-globulin-for-intravenous-injection therapy), or has not benefited much from splenectomy or has suffered from a postoperative recurrence.

In some embodiments of the present application, the treatment failure refers to ineffective treatment, or unsustainable efficacy, or recurrence.

In some embodiments, a patient with the immune thrombocytopenia may be treated with a combination of multiple therapies. In some embodiments of the present application, a patient with the immune thrombocytopenia may be treated with a combination of a first-line standard therapy, and/or a second-line standard therapy, and/or a third-line standard therapy, and/or a fourth-line standard therapy.

In some embodiments of the present application, a patient with the immune thrombocytopenia may be a patient who has previously received a glucocorticoid therapy and has been off medication for at least 3 weeks.

In some embodiments of the present application, a patient with the immune thrombocytopenia may be a patient who has previously received a danazol therapy and has been off medication for at least 4 weeks.

In some embodiments of the present application, a patient with the immune thrombocytopenia may be a patient who has previously received an azathioprine, cyclosporine (A), and/or mycophenolate mofetil therapy and has been off medication for at least 4 weeks.

In some embodiments of the present application, a patient with the immune thrombocytopenia may suffer from one or more (e.g., one, two, or three) types of immune thrombocytopenia. In some embodiments of the present application, the immune thrombocytopenia optionally includes a combination of any two or more of the types of immune thrombocytopenia described above. In some embodiments of the present application, a patient with the immune thrombocytopenia may suffer from two or more types of immune thrombocytopenia. For example, a patient with the immune thrombocytopenia may suffer from a combination of any two or more types of immune thrombocytopenia selected from the group consisting of the types of immune thrombocytopenia described above. As used in the present application, "has not benefited much" refers to, after treatment, a platelet count of less than 30 × 10⁹/L, or an increase of less than 2 times the baseline in platelet count, or the presence of bleeding.

### Administration Regimen

In some embodiments of the present application, the administration cycle of the compound of formula I or the pharmaceutically acceptable salt thereof, or the pharmaceutical composition thereof may be 2-6 weeks. In some embodiments of the present application, the administration cycle of the compound of formula I or the pharmaceutically acceptable salt thereof, or the pharmaceutical composition thereof may be 2 weeks, 3 weeks, 4 weeks, 5 weeks, 6 weeks, or a range formed by any of the aforementioned values. In some embodiments of the present application, the administration cycle of the compound of formula I or the pharmaceutically acceptable salt thereof, or the pharmaceutical composition thereof may be 4-5 weeks. In some embodiments, the administration cycle of the compound of formula I or the pharmaceutically acceptable salt thereof, or the pharmaceutical composition thereof may be 1-6 administration cycles, e.g., 1, 2, 3, 4, 5, or 6 administration cycles.

In some embodiments of the present application, the administration cycle for therapeutic treatment with the compound of formula I or the pharmaceutically acceptable salt thereof, or the pharmaceutical composition thereof may be 2-6 weeks. In some embodiments of the present application, the administration cycle for use in therapeutic treatment may be 2 weeks, 3 weeks, 4 weeks, 5 weeks, 6 weeks, or a range formed by any of the aforementioned values. In some embodiments of the present application, the administration cycle for use in therapeutic treatment may be 4-5 weeks.

In some embodiments of the present application, the compound of formula I or the pharmaceutically acceptable salt thereof, or the pharmaceutical composition thereof for use in treatment or prevention may be administered from once daily to 3 times daily, or once every two days; preferably once daily.

In some embodiments of the present application, the daily dose of the compound of formula I or the pharmaceutically acceptable salt thereof, or the pharmaceutical composition thereof may be selected from 50-1000 mg. In some embodiments of the present application, the daily dose of the compound of formula I or the pharmaceutically acceptable salt thereof, or the pharmaceutical composition thereof may be selected from the group consisting of 100 mg, 200 mg, 300 mg, 400 mg, 500 mg, 600 mg, 700 mg, 800 mg, 900 mg, 1000 mg, and a range formed by any of the aforementioned values. In some embodiments of the present application, the daily dose of the compound of formula I or the pharmaceutically acceptable salt thereof, or the pharmaceutical composition thereof is selected from the group consisting of 200-800 mg, 200-800 mg, 400-800 mg, 400-600 mg, and 600-800 mg.

In some embodiments of the present application, the daily dose of the compound of formula I or the pharmaceutically acceptable salt thereof, or the pharmaceutical composition thereof for use in treatment or prevention may be selected from 50-1000 mg. In some embodiments of the present application, the daily dose of the compound of formula I or the pharmaceutically acceptable salt thereof, or the pharmaceutical composition thereof for use in treatment or prevention may be selected from the group consisting of 100 mg, 200 mg, 300 mg, 400 mg, 500 mg, 600 mg, 700 mg, 800 mg, 900 mg, 1000 mg, and a range formed by any of the aforementioned values. In some embodiments of the present application, the daily dose of the compound of formula I or the pharmaceutically acceptable salt thereof, or the pharmaceutical composition thereof for use in treatment or prevention may be selected from the group consisting of 200-800 mg, 200-800 mg, 400-800 mg, 400-600 mg, and 600-800 mg.

In some embodiments of the present application, each dose of the compound of formula I or the pharmaceutically acceptable salt thereof, or the pharmaceutical composition thereof may be selected from 50-1000 mg. In some embodiments of the present application, each dose of the compound of formula I or the pharmaceutically acceptable salt thereof, or the pharmaceutical composition thereof may be selected from the group consisting of 50 mg, 100 mg, 200 mg, 300 mg, 400 mg, 500 mg, 600 mg, 700 mg, 800 mg, 900 mg, 1000 mg, and a range formed by any of the aforementioned values. In some embodiments of the present application, each dose of the compound of formula I or the pharmaceutically acceptable salt thereof, or the pharmaceutical composition thereof may be selected from the group consisting of 50-800 mg, 200-800 mg, 200-600 mg, 200-400 mg, 400-800 mg, 400-600 mg, and 600-800 mg.

In some embodiments of the present application, each dose of the compound of formula I or the pharmaceutically acceptable salt thereof, or the pharmaceutical composition thereof for use in treatment or prevention may be selected from 50-1000 mg. In some embodiments of the present application, each dose of the compound of formula I or the pharmaceutically acceptable salt thereof, or the pharmaceutical composition thereof for use in treatment or prevention may be selected from the group consisting of 50 mg, 100 mg, 200 mg, 300 mg, 400 mg, 500 mg, 600 mg, 700 mg, 800 mg, 900 mg, 1000 mg, and a range formed by any of the aforementioned values. In some embodiments of the present application, each dose of the compound of formula I or the pharmaceutically acceptable salt thereof, or the pharmaceutical composition thereof for use in treatment or prevention may be selected from the group consisting of 50-800 mg, 200-800 mg, 200-600 mg, 200-400 mg, 400-800 mg, 400-600 mg, and 600-800 mg.

In some embodiments of the present application, the compound of formula I or the pharmaceutically acceptable salt thereof is provided in the form of a pharmaceutical composition.

In some embodiments of the present application, in treating immune thrombocytopenia in a patient, the pharmaceutical composition is administered in a single dose once, twice, or three times daily. In some embodiments of the present application, in treating immune thrombocytopenia in a patient, the pharmaceutical composition is administered in a single dose of an oral solid formulation once, twice, or three times daily.

In some embodiments of the present application, in treating immune thrombocytopenia in a patient, the pharmaceutical composition is administered in multiple doses once or twice daily. In some embodiments of the present application, in treating immune thrombocytopenia in a patient, the pharmaceutical composition is administered in multiple doses of an oral solid formulation once or twice daily. In one specific embodiment, in treating immune thrombocytopenia in a patient, the pharmaceutical composition is administered in multiple doses of an oral solid formulation once daily.

In some embodiments of the present application, the pharmaceutical composition of the compound of formula I or the pharmaceutically acceptable salt thereof is in once-daily doses, with each dose being a single dose or multiple doses, preferably multiple doses, and the multiple doses consist of single doses of 10-200 mg of the compound of formula I or the pharmaceutically acceptable salt thereof. In some embodiments, the multiple doses consist of single doses of 10 mg, 20 mg, 40 mg, 50 mg, 60 mg, 80 mg, 100 mg, 120 mg, 150 mg, or 200 mg of the compound of formula I or the pharmaceutically acceptable salt thereof. In some embodiments of the present application, the pharmaceutical composition consists of a single dose of 50 mg or 200 mg of the compound of formula I or the pharmaceutically acceptable salt thereof.

In some embodiments of the present application, in treating immune thrombocytopenia in a patient, the compound of formula I or the pharmaceutically acceptable salt thereof is administered once daily at a dose of 200 mg, 400 mg, 600 mg, 800 mg, or a range formed by any of the aforementioned values for 28 days as one treatment cycle, and 5.6 g, 11.2 g, 16.8 g, 22.4 g, or a range formed by any of the aforementioned values of the compound of formula I or the pharmaceutically acceptable salt thereof is administered per treatment cycle.

In some embodiments of the present application, 28 days constitute one treatment cycle, in which the compound of formula I or the pharmaceutically acceptable salt thereof or the pharmaceutical composition thereof is administered consecutively from day 1 to day 28.

In some embodiments of the present application, 28 days constitute one treatment cycle, in which the compound of formula I or the pharmaceutically acceptable salt thereof or the pharmaceutical composition thereof is administered daily consecutively from day 1 to day 28.

In some embodiments of the present application, 28 days constitute one treatment cycle, in which the compound of formula I or the pharmaceutically acceptable salt thereof or the pharmaceutical composition thereof is administered once daily consecutively from day 1 to day 28.

In some embodiments of the present application, 28 days constitute one treatment cycle, in which the compound of formula I or the pharmaceutically acceptable salt thereof or the pharmaceutical composition thereof is administered on an empty stomach once daily consecutively from day 1 to day 28.

In some embodiments of the present application, 28 days constitute one treatment cycle, in which the compound of formula I or the pharmaceutically acceptable salt thereof or the pharmaceutical composition thereof is administered once daily consecutively from day 1 to day 28; each dose of the compound of formula I or the pharmaceutically acceptable salt thereof or the pharmaceutical composition thereof is a single dose, and the single dose is 50 mg or 200 mg of the compound of formula I or the pharmaceutically acceptable salt thereof.

In some embodiments of the present application, the compound of formula I or the pharmaceutically acceptable salt thereof or the pharmaceutical composition thereof may be packaged in a kit, and the kit comprises doses for one or more treatment cycles, or doses for administration within one treatment cycle, or doses in a range formed with any two of the foregoing values as endpoints of the compound of formula I or the pharmaceutically acceptable salt thereof or the pharmaceutical composition thereof. In some embodiments of the present application, the compound of formula I or the pharmaceutically acceptable salt thereof or the pharmaceutical composition thereof described above is packaged in a kit, and the kit comprises doses for 1-28 days (e.g., 1 day, 2 days, 3 days, 4 days, 5 days, 6 days, 7 days, 14 days, 21 days, or 28 days), or doses for one treatment cycle, two treatment cycles, or three treatment cycles or more, or doses in a range formed with any two of the foregoing values as endpoints of the compound of formula I or the pharmaceutically acceptable salt thereof or the pharmaceutical composition thereof described above.

In some embodiments of the present application, the compound of formula I or the pharmaceutically acceptable salt thereof or the pharmaceutical composition thereof is packaged in a kit, and the kit comprises doses for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, or 28 days, or doses in a range formed with any two of the foregoing values as endpoints of the compound of formula I or the pharmaceutically acceptable salt thereof or the pharmaceutical composition thereof described above. In some embodiments of the present application, the compound of formula I or the pharmaceutically acceptable salt thereof or the pharmaceutical composition thereof described above is packaged in a kit, and the kit comprises doses for 1 day, 2 days, 3 days, 4 days, 5 days, 6 days, 7 days, 14 days, 21 days, or 28 days, or doses in a range formed with any two of the foregoing values as endpoints of the compound of formula I or the pharmaceutically acceptable salt thereof or the pharmaceutical composition thereof described above.

In some embodiments of the present application, 28 days constitute one treatment cycle, in which a total dose of 5.6-22.5 g of the compound of formula **I** or the pharmaceutically acceptable salt thereof or the pharmaceutical composition thereof (calculated based on the active ingredient, the compound of formula **I**) is administered. In some embodiments of the present application, the total dose of the compound of formula **I** or the pharmaceutically acceptable salt thereof or the pharmaceutical composition thereof is selected from the group consisting of 5.6 g, 11.2 g, 16.8 g, 22.4 g, and a range formed by any of the aforementioned values (calculated based on the active ingredient, the compound of formula **I).** In some embodiments of the present application, the total dose of the compound of formula **I** or the pharmaceutically acceptable salt thereof or the pharmaceutical composition thereof is preferably 11.2-16.8 g (calculated based on the active ingredient, the compound of formula **I).** In some embodiments of the present application, the total dose of the compound of formula **I** or the pharmaceutically acceptable salt thereof or the pharmaceutical composition thereof is preferably 11.2 g or 16.8 g (calculated based on the active ingredient, the compound of formula **I).**

In some embodiments of the present application, the treatment cycle described above may be repeated as long as the disease is still under control and the administration regimen is clinically tolerable.

As used in the present application, the administration regimen described above is applicable to the compound of formula I or the pharmaceutically acceptable salt thereof or the pharmaceutical composition thereof, the pharmaceutical combination, or the kit described in the present application, or the use or method for treating or preventing immune thrombocytopenia described in the present application.

### Technical Effects

The compound of formula I of the present application has good safety in treating immune thrombocytopenia and can effectively improve the platelet count in patients with immune thrombocytopenia. The percentage of subjects with at least one platelet count of ≥30 × 10⁹/L and an increase of ≥2 times the baseline in platelet count within 12 weeks of treatment (in the absence of emergency treatment) is relatively high. The percentage of subjects with at least one platelet count of ≥50 × 10⁹/L within 12 weeks of treatment is also relatively high.

### Definitions and Explanations

Unless otherwise stated, the terms used herein shall have the following meanings. A certain term, unless otherwise specifically defined, should not be considered ambiguous or unclear, but construed according to its common meaning in the art. When referring to a trade name, it is intended to refer to its corresponding commercial product or its active ingredient.

As used herein, unless otherwise stated, the terms "include", "includes", "including", "comprise", "comprises", and "comprising" or equivalents thereof are open-ended statements and mean that elements, components, and steps that are not specified may be encompassed in addition to those listed.

All patents, patent applications, and other identified publications are explicitly incorporated herein by reference for the purpose of description and disclosure. Any reference to these publications herein shall not be construed as an admission that the publications form part of the commonly recognized knowledge in the art.

Unless otherwise specified, terms in the singular encompass those in the plural and vice versa. Unless otherwise specified, the word "a" or "an" means "at least one". Unless otherwise stated, "or" is used to mean "and/or".

The term "administer", "administration", or "administering" refers to physically introducing a therapeutic agent or a composition comprising a therapeutic agent to an entity using any of the many methods and delivery systems known to those skilled in the art. The terms "administer", "administration", and "administering" are used interchangeably herein.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable carrier and/or excipient" refers to those carriers and/or excipients that do not have a significant irritating effect on an organism and do not impair the biological activity and properties of the active compound.

The term "pharmaceutically acceptable salt" includes salts formed from basic radicals and free acids or salts formed from acidic radicals and free bases.

As used in the present application, "treatment failure" refers to a platelet count of <30 × 10⁹/L or an increase of <2 times the baseline in platelet count after 12 weeks of treatment.

As used in the present application, "unsustainable efficacy" means that a platelet count of <30 × 10⁹/L or an increase of <2 times the baseline in platelet count lasts for 12 weeks, with the exception of elevations in platelet count resulting from emergency treatment.

As used in the present application, the content of the compound of formula I or the pharmaceutically acceptable salt thereof, e.g., the amount administered, the dose, or the amount in the pharmaceutical composition, is calculated based on the free base form.

As used in the present application, if the compound in the pharmaceutical combination of the present application has, for example, at least one basic site, an acid addition salt may be formed. If needed, it may further form an acid addition salt with additional existing basic sites. A compound with at least one acidic group (for example, -COOH) can further form a salt with a base. A compound, for example, comprising both carboxyl and amino, can further form an inner salt.

The term "patient" is a mammal, such as a human, dog, cow, horse, pig, sheep, goat, cat, mouse, rabbit, rat, or transgenic non-human animal. In some embodiments, the patient is a human. As used herein, the terms "subject" and "patient" are used interchangeably.

The term "pharmaceutical composition" refers to a mixture consisting of one or more of the compounds or the salts thereof of the present application and a pharmaceutically acceptable auxiliary material. The pharmaceutical composition is intended to facilitate the administration of the compound or the salt thereof of the present application to a patient.

The term "treatment" generally refers to obtaining desired pharmacological and/or physiological effects, including partially or completely stabilizing or curing a disease and/or an effect that the disease has. As used herein, "therapy", "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "prevent" or "prevention" means administering the compound or formulation of the present disclosure to prevent a disease or one or more symptoms associated with the disease, including: preventing the occurrence of the disease or disease state in a mammal, particularly when such a mammal is predisposed to the disease state but has not yet been diagnosed with it.

The term "effective amount" refers to an amount of the compound of the present application for (i) treating a specific disease, condition, or disorder, or (ii) alleviating, ameliorating, or eliminating one or more symptoms of a specific disease, condition, or disorder. The amount of the compound of the present application composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "single dose" or "single-dose" refers to the smallest unit of packaging containing a certain quantity of a pharmaceutical product; for example, in a box of seven capsules, each capsule is a single dose; or a vial of injection is a single dose. The term "multiple doses" consists of multiple single doses.

The term "recurrent" means that a disease recurs after an objective response is achieved through treatment with a certain treatment regimen.

The term "refractory" means that a particular disease is resistant or non-responsive to therapy with a particular therapeutic agent. For example, refractory immune thrombocytopenia is defined as a diagnosis of immune thrombocytopenia confirmed by diagnostic re-evaluation in a patient who has failed to respond to first-line therapeutic agents and second-line thrombopoietic drugs and rituximab or who has failed to respond to splenectomy/suffered from a postoperative recurrence.

### DETAILED DESCRIPTION

The present invention is illustrated in more detail through specific examples. The following examples are provided for illustrative purposes only and are not intended to limit the present invention in any way.

The compound of formula I was prepared based on the method disclosed in WO2018228475.

### Example 1 Clinical Trial for Immune Thrombocytopenia

### 1.1 Test drug and administration regimen

Test drug: tablets of the compound of formula I, 50 mg/tablet or 200 mg/tablet;
Administration regimen: orally taken once daily on an empty stomach, with 28 consecutive days of administration as one treatment cycle.

### 1.2 Enrollment criteria

1) The subject volunteers to participate in this study, needs to sign a consent form, and is expected to have a survival period of more than 3 months.
2) The subject is a male or female aged 18-75 (inclusive) (at the time of signing the consent form) and has a physical condition score (American Eastern Cooperative Oncology Group [ECOG] score) of 0-2.
3) The subject was diagnosed with persistent or chronic immune thrombocytopenia at least 3 months prior to screening (platelet count has been continuously decreasing for more than 6 months).
4) The subject has previously failed at least one first-line standard therapy for immune thrombocytopenia (glucocorticoid and/or intravenous gamma globulin) (no response or unsustainable efficacy or recurrence), or has not benefited much from splenectomy or has suffered from a postoperative recurrence.
5) The subject has a WHO bleeding score of 0-1 and is expected to not require emergency treatment within 2 weeks after enrollment according to the judgment of the researchers.
6) The subject had a mean value of at least 2 independent platelet counts (over 24 h apart) within 2 months prior to screening of <30 × 10⁹/L, with each of the platelet counts being ≤35 × 10⁹/L; and experienced no severe bleeding within 4 weeks prior to screening.
7) Main organs function well and the following criteria should be met:
   a. Neutrophil count (ANC) > 1.5 × 10⁹/L; hemoglobin (Hb) > 90 g/L;
   b. Total bilirubin (TBIL), alanine aminotransferase (ALT), and aspartate aminotransferase (AST) ≤ 1.5 times the upper limit of normal (ULN); serum creatinine ≤ 1.5 × ULN, and creatinine clearance rate ≥ 50 mL/min;
   c. Serum amylase and lipase do not exceed the upper limits of normal;
   d. Prothrombin time (PT) is within the normal range ± 3 s, and activated partial thromboplastin time (APTT) is within the normal range ± 10 s;
   e. The subject has no history of coagulation disorders other than immune thrombocytopenia.
8) A female subject of childbearing age should agree to take contraceptive measures (such as intrauterine devices, contraceptives, or condoms) during the study and for 90 days after the study; serum pregnancy test results should be negative within 7 days before enrollment, and the subject must be a non-lactating one; a male subject should agree to take contraceptive measures during the study and for 6 months after the study.

### 1.3 Evaluation method and measure

Efficacy was evaluated according to the platelet counts, WHO bleeding scores, and emergency treatment received of the subjects. The effective rate of platelet count increase was used as a main measure of efficacy, and for evaluation criteria, reference was made to *American Society of Hematology 2019 Guidelines for Immune Thrombocytopenia* and *Chinese Guidelines for Diagnosing and Treating Adult Primary Immune Thrombocytopenia (2020 Edition).*

### 1.4 Experimental results

### 1.4.1 Safety

The overall safety of the tablets of the compound of formula I in the adult subjects with persistent or chronic immune thrombocytopenia was controllable, with no dose-limiting toxicity (DLT) events and no treatment-related adverse events of grade 3 or higher.

### 1.4.2 Efficacy

A total of 6 subjects were enrolled, with 3 in the 400 mg group (the dose for 2 of them was increased to 600 mg) and 3 in the 600 mg group.

The percentage of subjects with at least one platelet count of ≥30 × 10⁹/L and an increase of ≥2 times the baseline in platelet count within 12 weeks of treatment among the evaluated patients (in the absence of emergency treatment) was 50%; the percentage of subjects with at least one platelet count of ≥50 × 10⁹/L within 12 weeks of treatment was 33.3%. The results show that the tablets of the compound of formula I of the present application have excellent clinical efficacy in treating adult persistent or chronic immune thrombocytopenia.

Table 1 shows the efficacy in representative cases.

**Table 1. Efficacy in representative cases**

| Subject No. | History of previous therapy | Dose group | Mean value of baseline platelet counts (× 10⁹/L) | Evaluation of efficacy (platelet count (× 10⁹/L)) | | | | | | | | | | | | |
|---|---|---|---|---|---|---|---|---|---|---|---|---|---|---|---|---|
| | | | | D3 | D11 | D18 | D25 | W5D1 | W6D1 | W7D1 | W8D1 | W9D1 | W10D1 | W11D1 | W12D1 | W13D1 |
| 1 | Third-line therapy | 600mg/d | 22 | 52 | 220 | 7 | 9 | 89 | 84 | 35 | 131 | 224 | 25 | 48 | 100 | -- |
| 2 | Second-line therapy | 600mg/d | 6.5 | 10 | 31 | 35 | 23 | -- | -- | -- | -- | -- | -- | -- | -- | -- |
| 3 | First-line therapy | 600mg/d | 33.5 | 58 | 83 | 34 | -- | -- | -- | -- | -- | -- | -- | -- | -- | -- |

| | | | | | | | | | | | | | | | | |
|---|---|---|---|---|---|---|---|---|---|---|---|---|---|---|---|---|
| Note: "--" indicates that data have not yet been obtained. | | | | | | | | | | | | | | | | |

## Claims

1. A compound of formula I or a pharmaceutically acceptable salt thereof, for use in treating or preventing immune thrombocytopenia:

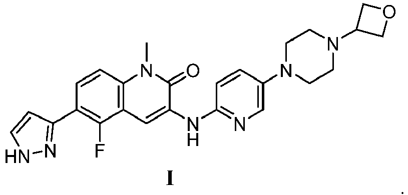

2. The compound of formula I or the pharmaceutically acceptable salt thereof according to claim 1, wherein the immune thrombocytopenia is primary immune thrombocytopenia or secondary immune thrombocytopenia; preferably, the immune thrombocytopenia is primary immune thrombocytopenia.

3. The compound of formula I or the pharmaceutically acceptable salt thereof according to claim 1, wherein the immune thrombocytopenia is persistent or chronic immune thrombocytopenia; preferably, the immune thrombocytopenia is persistent or chronic primary immune thrombocytopenia; more preferably, the immune thrombocytopenia is adult persistent or chronic primary immune thrombocytopenia.

4. The compound of formula I or the pharmaceutically acceptable salt thereof according to claim 1, wherein the immune thrombocytopenia is recurrent and/or refractory primary immune thrombocytopenia.

5. The compound of formula I or the pharmaceutically acceptable salt thereof according to claim 3, wherein a patient with the persistent or chronic immune thrombocytopenia is a patient who has been undergoing a persistent decrease in platelet count for more than 3 months following diagnosis; alternatively, a patient with the persistent or chronic immune thrombocytopenia is a patient who has been undergoing a persistent decrease in platelet count for more than 6 months following diagnosis.

6. The compound of formula I or the pharmaceutically acceptable salt thereof according to claim 1, wherein a patient with the immune thrombocytopenia is a patient who has previously received one or more prior therapies.

7. The compound of formula I or the pharmaceutically acceptable salt thereof according to claim 6, wherein the prior therapies include a drug therapy or a surgical therapy; optionally, the drug therapy includes a glucocorticoid therapy, an immunoglobulin therapy, a molecularly targeted therapy, a thrombopoietic drug therapy, or an immunosuppressant therapy, and the surgical therapy is a splenectomy therapy.

8. The compound of formula I or the pharmaceutically acceptable salt thereof according to claim 7, wherein drugs for use in the drug therapy are selected from the group consisting of: prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, immunoglobulins for injection, rituximab, veltuzumab, daclizumab, basiliximab, ocrelizumab, alemtuzumab, avatrombopag, eltrombpag, romiplostim, lusutrombopag, recombinant human thrombopoietin (rhTPO), danazol, azathioprine, cyclosporine (A), vincristine, mycophenolate mofetil, all-trans retinoic acid, and decitabine, or a combination of one or more of the drugs described above.

9. The compound of formula I or the pharmaceutically acceptable salt thereof according to any one of claims 1 to 8, wherein the patient with the immune thrombocytopenia is a patient who has previously failed one or more first-line or multi-line standard therapies; optionally, the patient with the immune thrombocytopenia is a patient who has previously failed one or more first-line, second-line, or third-line standard therapies; further optionally, the patient with the immune thrombocytopenia is a patient who has previously failed one, two, three, four, or five first-line standard therapies.

10. The compound of formula I or the pharmaceutically acceptable salt thereof according to any one of claims 1 to 9, wherein one treatment cycle for treating or preventing the immune thrombocytopenia is 2-6 weeks.

11. The compound of formula I or the pharmaceutically acceptable salt thereof according to any one of claims 1 to 10, wherein an administration regimen for treating or preventing the immune thrombocytopenia is from once daily to 3 times daily, or once every two days.

12. The compound of formula I or the pharmaceutically acceptable salt thereof according to any one of claims 1 to 11, wherein a daily dose for treating or preventing the immune thrombocytopenia is selected from 50-1000 mg.

13. The compound of formula I or the pharmaceutically acceptable salt thereof according to any one of claims 1 to 12, wherein each dose for treating or preventing the immune thrombocytopenia is selected from 50-1000 mg.

14. A pharmaceutical composition for use in treating or preventing immune thrombocytopenia in a subject in need thereof, wherein the pharmaceutical composition comprises the compound of formula I or the pharmaceutically acceptable salt thereof according to any one of claims 1 to 13; optionally, the pharmaceutical composition comprises a single dose of 10-200 mg of the compound of formula I or the pharmaceutically acceptable salt thereof according to any one of claims 1 to 13.

15. Use of a compound of formula I or a pharmaceutically acceptable salt thereof, or a pharmaceutical composition thereof, for preparing a medicament for treating or preventing immune thrombocytopenia in a subject in need thereof.
